# EUROPEAN PATENT APPLICATION

(11) **EP 3 066 986 A1**
(43) Date of publication of application: **14.09.2016**
(21) Application number: 14860520.7
(22) Date of filing: 22.10.2014
(51) Int. Cl.: A61B 10/00, A61B 1/00, A61B 5/00, A61B 5/15, A61B 10/04, G01N 1/10

(54) **BODY-FLUID SAMPLING DEVICE**

(30) Priority: 06.11.2013 JP 2013230309
(71) Applicant: OLYMPUS CORPORATION, Hachioji-shi Tokyo 192-8507 (JP)
(72) Inventor: SUZUKI Satoko, Tokyo 192-8507 (JP); SAKAMOTO Hiroko, Tokyo 192-8507 (JP); MIKKAICHI Takayasu, Tokyo 192-8507 (JP); AIKAWA Yoshie, Tokyo 192-8507 (JP); HASHIMOTO Tatsutoshi, Tokyo 192-8507 (JP)
(74) Representative: Schicker, Silvia
(86) International application number: PCT/JP2014/078085
(87) International publication number: WO 2015/068575

(57) **Abstract**

A body fluid collection device includes a long shaft member having a lumen formed along a longitudinal axis, an absorption body configured to expand from an initial state in which a body fluid is not absorbed into the absorption body as the body fluid is absorbed, and having a proximal end portion configured to be disposed in the lumen when a predetermined amount of the body fluid is absorbed into the absorption body, and an identification section for identifying that the absorption body has absorbed the predetermined amount of the body fluid.

## Description

### Technical Field

The present invention relates to a body fluid collection device inserted into a living body to collect a body fluid.

Priority is claimed on Japanese Patent Application No. 2013-230309, filed November 6, 2013, the content of which is incorporated herein by reference.

### Background Art

In the related art, a body fluid collection device is introduced into a living body to collect a body fluid, and tests are performed on the living body using the body fluid. For example, a body fluid collection tool disclosed in Patent Literature 1 includes an elongated catheter (a long shaft member) having flexibility, and a movable absorption body unit inserted through a hollow hole of the catheter so as to advance and retract. The movable absorption body unit is constituted by mechanically connecting a shaft-shaped member disposed at a distal end side and formed of a semi-hard material and a relatively flexible manipulation wire disposed at a proximal end side via a joint. An absorption body constituted by a liquid absorption material is fixed to a distal end of the shaft-shaped member. The absorption body has a distal end portion formed in a substantially spherical shape and a rear end portion formed in a spindle shape that gradually thins.

The body fluid collection tool having the afore-mentioned configuration is inserted through a channel of the endoscope to be guided to a target region. When the body fluid is collected at the target region, the entire absorption body protrudes from the distal end of the catheter.

### Citation List

### Patent Literature

Patent Literature 1: Japanese Unexamined Patent Application, First Publication No. 2001-137248

### Summary of Invention

### Technical Problem

However, in the body fluid collection tool disclosed in Patent Literature 1, when the absorption body is accommodated in the catheter in order to recover the absorption body that has collected the body fluid, the body fluid absorbed into the absorption body is squeezed out of the absorption body. For this reason, there is a problem that the amount of the body fluid absorbed into the absorption body upon recovery is not constant.

In consideration of the above-mentioned problems, the present invention is directed to provide a body fluid collection device capable of easily recovering an absorption body in a state in which a predetermined amount of body fluid is absorbed into the absorption body.

### Solution to Problem

According to a first aspect of the present invention, a body fluid collection device includes: a long shaft member having a lumen formed along a longitudinal axis; an absorption body configured to expand from an initial state in which a body fluid is not absorbed into the absorption body as the body fluid is absorbed, and having a proximal end portion configured to be disposed in the lumen when a predetermined amount of the body fluid is absorbed into the absorption body; and an identification section for identifying that the absorption body has absorbed the predetermined amount of the body fluid.

According to a second aspect of the present invention, in the body fluid collection device according to the first aspect of the present invention, the identification section may be an obscured glass provided at the long shaft member such that the obscured glass is observable from an outer circumferential surface side, and a surface of the obscured glass disposed at an inner circumferential surface side of the long shaft member may be an obscured machined surface. The absorption body may have an outer diameter equal to or smaller than an inner diameter of the lumen in the initial state, and may be disposed more distal than the obscured machined surface. When the absorption body has absorbed the predetermined amount of the body fluid, the outer diameter of the absorption body may become equal to the inner diameter of the lumen, and a proximal end of the absorption body may extend to be closer to a proximal end of the long shaft member than a distal end of the obscured machined surface.

According to a third aspect of the present invention, in the body fluid collection device according to the second aspect of the present invention, the absorption body may be attached to a distal end portion of a manipulation member inserted into the lumen such that the manipulation member is movable along the longitudinal axis. When the manipulation member is moved to the distal end side with respect to the long shaft member, a distal end portion of the absorption body may be disposed more distal than the long shaft member.

According to a fourth aspect of the present invention, in the body fluid collection device according to the first aspect of the present invention, the identification section may be a pigment of Commelina communis provided at an inner circumferential surface side of the long shaft member such that the pigment is observable from an outer circumferential surface side of the long shaft member. The absorption body may be disposed more distal than the pigment in the initial state. When the absorption body has absorbed the predetermined amount of the body fluid, an outer diameter of the absorption body may become equal to an inner diameter of the lumen, and a proximal end of the absorption body may extend to be closer to a proximal end of the long shaft member than a distal end of an area in which the pigment is provided.

According to a fifth aspect of the present invention, in the body fluid collection device according to the first aspect of the present invention, the identification section may be a side hole formed to reach the lumen from an outer circumferential surface of the long shaft member. The absorption body may not block the side hole in the initial state, and when the absorption body has absorbed the predetermined amount of the body fluid, the expanded absorption body may block the side hole.

According to a sixth aspect of the present invention, in the body fluid collection device according to the first aspect of the present invention, the identification section may be a side hole formed to reach the lumen from an outer circumferential surface of the long shaft member. The absorption body may have an outer diameter smaller than an inner diameter of the lumen in the initial state, and may be disposed inside the lumen to be spaced from the side hole and disposed radially inward with respect to the side hole. When the absorption body has absorbed the predetermined amount of the body fluid, the absorption body may expand radially outward such that the outer diameter of the absorption body becomes equal to the inner diameter of the lumen.

According to a seventh aspect of the present invention, in the body fluid collection device according to the first aspect of the present invention, the absorption body may have an outer circumferential surface in contact with an inner circumferential surface of the lumen throughout an entire circumference of the absorption body. The identification section may be a communication hole formed in the absorption body along the longitudinal axis. When the absorption body has absorbed the predetermined amount of the body fluid, an inner circumferential surface of the communication hole of the absorption body may expand radially inward by a radius of the communication hole.

According to an eighth aspect of the present invention, in the body fluid collection device according to the first aspect of the present invention, the identification section may have: a pair of electrodes provided on an inner circumferential surface of the lumen to face each other; and a light generating section, a sound generating section, or a vibration generating section connected to each of the electrodes and configured to be operated when a closed circuit is formed together with the electrodes. The absorption body may have an outer diameter equal to or smaller than a distance between the electrodes in the initial state. In the initial state, the absorption body may not be disposed between the electrodes. When the absorption body has absorbed the predetermined amount of the body fluid, the expanded absorption body may be disposed between the electrodes, and the outer diameter of the absorption body may be increased to be equal to or larger than the distance between the electrodes.

### Advantageous Effects of Invention

According to the body fluid collection device of the above-mentioned aspects, the absorption body can be easily recovered in a state in which a predetermined amount of body fluid is absorbed into the absorption body.

### Brief Description of Drawings

Fig. 1 is a side cross-sectional view of a body fluid collection device according to a first embodiment of the present invention.
Fig. 2 is a general view showing an endoscope used in combination with the body fluid collection device, a portion of which is cut away.
Fig. 3 is a view showing a sequence of collecting duodenal juice using the body fluid collection device.
Fig. 4 is a view showing the sequence of collecting the duodenal juice using the body fluid collection device.
Fig. 5 is a cross-sectional view of a major part in a modified example of the body fluid collection device according to the first embodiment of the present invention.
Fig. 6 is a cross-sectional view of the major part showing an action of the body fluid collection device.
Fig. 7 is a cross-sectional view of the major part in a modified example of the body fluid collection device according to the first embodiment of the present invention.
Fig. 8 is a cross-sectional view of the major part showing an action of the body fluid collection device.
Fig. 9 is a cross-sectional view of a major part in a modified example of the body fluid collection device according to the first embodiment of the present invention.
Fig. 10 is a cross-sectional view of the major part showing an action of the body fluid collection device.
Fig. 11 is a side cross-sectional view of a body fluid collection device according to a second embodiment of the present invention.
Fig. 12 is a side cross-sectional view showing an action of the body fluid collection device.
Fig. 13 is a cutaway perspective view of a major part of a body fluid collection device according to a third embodiment of the present invention.
Fig. 14 is a cutaway perspective view of the major part showing an action of the body fluid collection device.
Fig. 15 is a side cross-sectional view of a body fluid collection device according to a fourth embodiment of the present invention.
Fig. 16 is a side cross-sectional view showing an action of the body fluid collection device.

### Description of Embodiments

### (First embodiment)

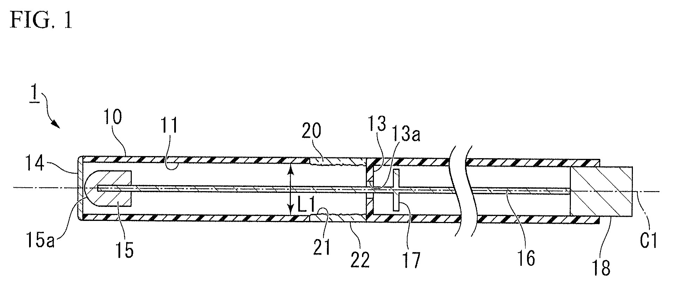

Hereinafter, a body fluid collection device (hereinafter, also simply referred to as a "device") according to a first embodiment of the present invention will be described with reference to Figs. 1 to 10.

As shown in Fig. 1, a device 1 according to the embodiment includes a flexible sheath (a long shaft member) 10 having a lumen (a through-hole) 11 formed along a longitudinal axis C1, an absorption body 15 configured to expand by absorbing a body fluid, and an obscured glass (an identification section) 20 configured to identify that the absorption body 15 has absorbed a predetermined amount of body fluid.

The sheath 10 is formed in a cylindrical shape. A locking section 13 protruding from an inner circumferential surface of the lumen 11 inward in the radial direction of the sheath 10 is formed at the sheath 10 throughout the entire circumference of the sheath 10. For this reason, a transmission hole 13a having a substantially circular shape when seen in a direction along the longitudinal axis C1 is formed at the locking section 13. The sheath 10 is formed of a resin such as polytetrafluoroethylene (PTFE) or the like to have a certain strength with respect to a tensile force along the longitudinal axis C1 and a force spreading outward in the radial direction. A protection film 14 configured to block an opening of a distal end portion of the lumen 11 is attached to the sheath 10. The protection film 14 may be formed of, for example, a porous film of a PTFE resin or the like. The protection film 14 is set to a thickness such that the protection film 14 can be broken by being pressed with the absorption body 15 while preventing the body fluid from entering the lumen 11 as will be described below.

The obscured glass 20 is provided to pass through the sheath 10 from the inner circumferential surface to an outer circumferential surface of the sheath 10 at a position that is in the vicinity of the locking section 13 and closer to the distal end side than the locking section 13 in the sheath 10. That is, the obscured glass 20 is provided at the sheath 10 so as to be observable from the outer circumferential surface side. The obscured glass 20 may be provided throughout the entire circumference of the sheath 10 or may be provided at a portion in the circumferential direction of the sheath 10. The obscured glass 20 is provided at a distal end side far from the locking section 13 by a predetermined length. An obscured machined surface 21 having a fine uneven shape formed at an inner circumferential surface side of the sheath 10 is formed at the obscured glass 20. The obscured machined surface 21 appears opaque because light (visible rays) is scattered in a state in which no matter is attached to the obscured machined surface 21. The outer circumferential surface of the obscured glass 20 is a mirror surface 22 formed to be flatter than the obscured machined surface 21. The inner circumferential surface and the outer circumferential surface of the obscured glass 20 and the sheath 10 of the distal end side of the obscured glass 20 are flush with each other. The obscured glass 20 and the locking section 13 are disposed in parallel with no gap there between in the direction along the longitudinal axis C1.

The absorption body 15 is formed of polyvinyl acetate or the like in a columnar shape. In an initial state in which the body fluid is not absorbed, the outer diameter of the absorption body 15 is equal to or smaller than an inner diameter L1 of the lumen 11. A distal end surface 15a of the absorption body 15 is formed in a curved shape to become convex toward the distal end side. In the absorption body 15, even when the body fluid is absorbed from a portion of the absorption body 15, the body fluid spreads throughout the absorption body 15 due to a capillary phenomenon. The absorption body 15 expands as the body fluid is absorbed from the initial state. In the example, the absorption body 15 of the device 1 before use is disposed in the lumen 11. The absorption body 15 is attached to a distal end portion of a support rod (a manipulation member) 16 as the support rod 16 extending along the longitudinal axis C1 is inserted from the proximal end side. The outer diameter of the support rod 16 is smaller than the inner diameter of the transmission hole 13a of the locking section 13. The distal end portion of the support rod 16 is fixed in the absorption body 15. A locked section 17 protruding outward in the radial direction is formed at a portion of the support rod 16 closer to the proximal end side than the locking section 13 throughout in the circumferential direction of the sheath 10. The outer diameter of the locked section 17 is smaller than the above-mentioned inner diameter L1 and larger than the inner diameter of the transmission hole 13a. The support rod 16 and the locked section 17 are integrally formed of a metal having biocompatibility such as stainless steel. The support rod 16 is movably inserted through the lumen 11 of the sheath 10 along the longitudinal axis C1.

A columnar handle 18 is fixed to a proximal end portion of the support rod 16. The outer diameter of the handle 18 is smaller than the inner diameter L1.

When the entire absorption body 15 is accommodated in the lumen 11, the locked section 17 of the support rod 16 is disposed to be separate from the locking section 13 toward the proximal end. The distance between the locking section 13 and the locked section 17 is set such that the proximal end portion of the absorption body 15 is disposed in the lumen 11 when the absorption body 15 absorbs a predetermined amount of body fluid in a state in which the locked section 17 is locked to the locking section 13 by pushing the support rod 16 as will be described below.

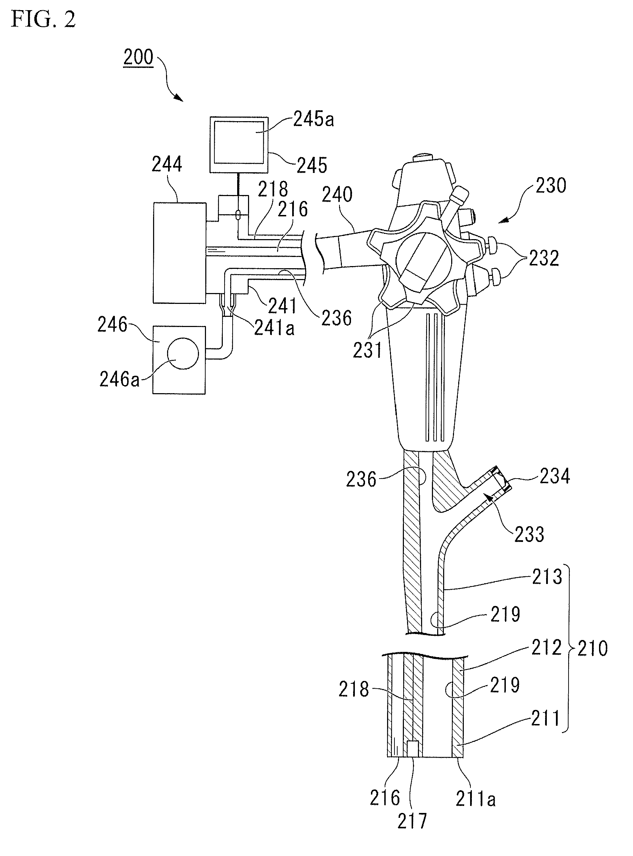

The device 1 having the above-mentioned configuration is used in combination with a known endoscope 200 shown in Fig. 2. The endoscope 200 is a so-called direct viewing type endoscope, and includes an elongated insertion section 210, a manipulation unit 230 provided at a proximal end portion of the insertion section 210, and a universal cable 240 having one end portion attached to a side surface of the manipulation unit 230. The insertion section 210 has a distal end hard section 211 provided at a distal end thereof, a curved section 212 attached to a proximal end portion of the distal end hard section 211 and configured to be curved by manipulation, and a flexible tube section 213 attached to a proximal end portion of the curved section 212 and having flexibility.

A light guide 216 constituted by a bundle of optical fibers and an observation unit 217 having an object lens and a charge coupled device (CCD) are provided at a distal end surface 211a of the distal end hard section 211 in a state in which they are exposed to the outside. The light guide 216 is inserted through the insertion section 210 and the manipulation unit 230 to extend to the universal cable 240. The object lens images an image of an observation target in front of the distal end hard section 211 on a light receiving surface of the CCD. The CCD converts the image of the observation target into a signal representing an image, and transmits the converted signal to a signal wiring 218. The signal wiring 218 is inserted through the insertion section 210 and the manipulation unit 230 to extend to the universal cable 240. An opening in communication with a channel 219 is formed in the distal end surface 211 a of the distal end hard section 211. The proximal end side of the channel 219 extends to the manipulation unit 230 through the flexible tube section 213.

In the curved section 212, while not shown, a plurality of curved pieces are connected in a longitudinal direction of the insertion section 210. A distal end portion of a manipulation wire is attached to the curved piece of the most distal end side. The proximal end side of the manipulation wire extends to the manipulation unit 230 through the flexible tube section 213.

A knob 231 configured to pull or push the manipulation wire, and a switch 232 configured to manipulate an opening/closing valve or the like (not shown) provided in the manipulation unit 230 is provided at the manipulation unit 230. As the knob 231 is manipulated, the manipulation wire can be manipulated to curve the curved section 212 in a desired direction. An insertion port 233 in communication with the channel 219 is provided at the distal end side of the manipulation unit 230. A forceps port 234 is attached to the insertion port 233. In the manipulation unit 230, a distal end portion of a suction conduit 236 communicates with the channel 219. The proximal end side of the suction conduit 236 extends into the universal cable 240.

An integrated connector 241 is provided at the proximal end side of the universal cable 240. A light source device 244 and a monitor 245 are attached to the integrated connector 241. The light source device 244 supplies illumination light emitted from a built-in lamp (not shown) to a proximal end surface of the light guide 216. A signal processing circuit (not shown) is provided in the monitor 245. A signal transmitted through the signal wiring 218 is converted by the signal processing circuit, and the image of the observation target acquired by the CCD is displayed on a display panel 245a of the monitor 245.

A suction device 246 is connected to a connector 241a provided at the integrated connector 241. A pump 246a configured to perform a suction operation is provided in the suction device 246. The suction device 246 connected to the connector 241 a can suction the inside of the channel 219 via the suction conduit 236 as the pump 246a is operated when the above-mentioned opening/closing valve is open.

Next, an action when the device 1 having the above-mentioned configuration is used in combination with the endoscope 200 will be described in the case in which the duodenal juice (the body fluid) is collected by the device 1. Further, the duodenal juice referred to herein includes not only duodenal juice secreted from the duodenum in a narrow sense but also pancreatic juice and bile.

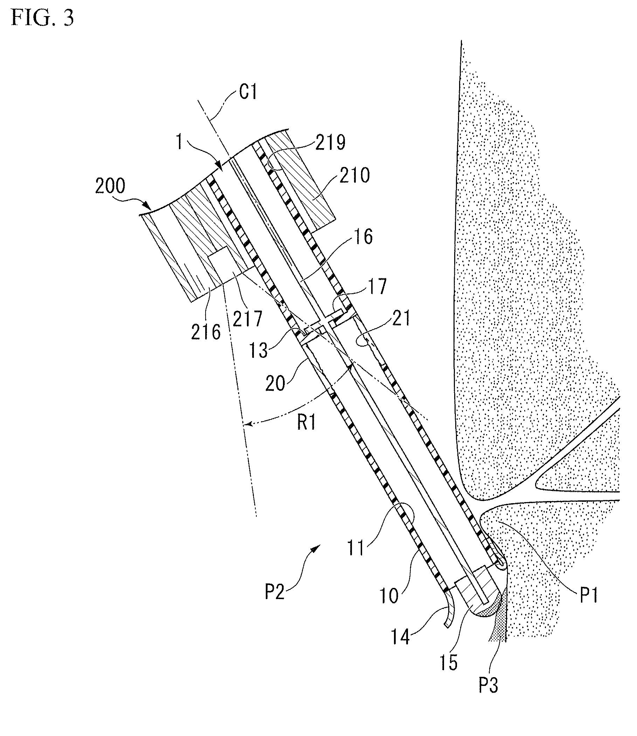

First, a user such as an operator or the like illuminates the illumination light emitted from the light source device 244 to the front of the insertion section 210 via the light guide 216. The insertion section 210 of the endoscope 200 is inserted into the body of the patient from a natural opening such as the mouth, and as shown in Fig. 3, the insertion section 210 advances to the duodenum P2 in the vicinity of the duodenal papilla P1. When the insertion section 210 is inserted, the knob 231 is manipulated to appropriately curve the curved section 212 if necessary while the picture displayed on the display panel 245a of the monitor 245 is observed. In addition, as the switch 232 is manipulated to suction the inside of the channel 219, a physiological saline solution or the like (not shown) used to clean the inside of the duodenum P2 is suctioned out of the body.

The sheath 10 of the device 1 is inserted into the channel 219 from the distal end side through the forceps port 234. The distal end portion of the sheath 10 protrudes from the channel 219. The support rod 16 is moved (pushed) to the distal end side with respect to the sheath 10 via the handle 18, and the locked section 17 is locked to the surface of the proximal end side of the locking section 13. Here, the distal end portion of the absorption body 15 is disposed closer to the distal end side than the sheath 10, and the protection film 14 is torn by being pressed with the absorption body 15. The proximal end portion of the absorption body 15 is disposed closer to the distal end side of the lumen 11 than the obscured machined surface 21 of the obscured glass 20. That is, in this state, the absorption body 15 does not come in contact with the obscured machined surface 21. The protrusion amount of the sheath 10 from the channel 219 is adjusted such that the obscured glass 20 enters a field of vision range R1 corresponding to the picture acquired by the CCD of the observation unit 217.

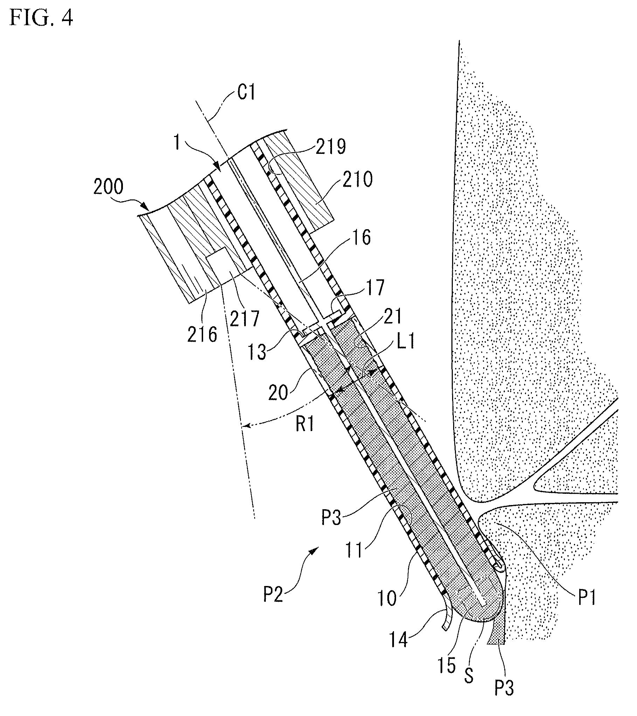

As the knob 231 is manipulated to curve the curved section 212 or the like, the absorption body 15 comes in contact with the duodenal juice P3 in the duodenum P2. As the distal end portion of the absorption body 15 is disposed closer to the distal end side than the sheath 10, the absorption body 15 can easily come in contact with the duodenal juice P3. The duodenal juice P3 is absorbed into the absorption body 15. The user observes the picture of the obscured glass 20 using the monitor 245 while holding a state in which the locked section 17 is locked to the locking section 13 to position the absorption body 15 with respect to the sheath 10. As shown in Fig. 4, the absorption body 15 extends toward the proximal end side while absorbing the duodenal juice P3 such that the shape expands from the original shape S and the outer diameter increases. When a predetermined amount of the duodenal juice P3 is absorbed, the outer diameter of the proximal end side of the absorption body 15 becomes equal to the inner diameter L1 of the lumen 11, and the proximal end of the absorption body 15 extends closer to the proximal end side than the distal end of the obscured machined surface 21 to come in contact with the locking section 13. The predetermined amount disclosed herein is, for example, about 50 µL (microliters, 10⁻⁹ m³) to 53 µL. The proximal end portion of the absorption body 15 comes in contact with the obscured machined surface 21. The obscured machined surface 21 is changed such that an effect of scattering the light is weakened by moisture or the like included in the duodenal juice P3 and a color of the obscured glass 20 is changed from opaque toward transparent as the duodenal juice P3 absorbed to the absorption body 15 is attached to the obscured machined surface 21. As the proximal end portion of the absorption body 15 that has absorbed the duodenal juice P3 comes in contact with the locking section 13, the duodenal juice P3 is reliably prevented from being further absorbed from a state in which the duodenal juice P3 is absorbed until the absorption body 15 comes in contact with the obscured glass 20.

The user moves (pulls) the device 1 to the proximal end side with respect to the forceps port 234 and extracts the device 1 from the channel 219 of the endoscope 200 after checking a variation in color of the obscured glass 20 through the monitor 245. As the proximal end portion of the absorption body 15 that has absorbed the duodenal juice P3 is disposed in the lumen 11, the device 1 is extracted from the channel 219 in a state in which the proximal end portion of the absorption body 15 that has absorbed the predetermined amount of the duodenal juice P3 is covered by the sheath 10. Next, the insertion section 210 of the endoscope 200 is extracted from the mouth of the patient. In the device 1 that has absorbed the predetermined amount of the duodenal juice P3, for example, the absorption body 15 is removed from the device 1, and the duodenal juice P3 absorbed into the absorption body 15 is extracted in a diluted solution. A concentration of a specific tumor marker in the pancreatic juice extracted in the diluted solution is measured, and a test for pancreatic cancer or the like is performed.

As described above, according to the device 1 of the embodiment, since the proximal end portion of the absorption body 15 is disposed in the lumen 11 when the absorption body 15 absorbs the duodenal juice P3, a side surface of the proximal end portion of the absorption body 15 that expands has a constant shape. Accordingly, the predetermined amount of the duodenal juice P3 can be precisely absorbed by the absorption body 15. Since the obscured glass 20 serving as an identification section is provided, by observing the variation in color of the obscured glass 20 in contact with the absorption body 15 that has absorbed the duodenal juice P3 with the endoscope 200, the user can easily identify that the absorption body 15 has absorbed the predetermined amount of the duodenal juice P3. As the proximal end portion of the absorption body 15 that has absorbed the duodenal juice P3 is disposed in the lumen 11, the proximal end portion of the absorption body 15 that has absorbed the duodenal juice P3 can be prevented from coming in contact with the inner circumferential surface or the like of the channel 219, and the absorption body 15 can be easily recovered in a state in which the predetermined amount of the duodenal juice P3 is absorbed into the absorption body 15.

Since absorption of the predetermined amount of the duodenal juice P3 into the absorption body 15 can be identified, any user can easily collect the predetermined amount of the duodenal juice P3 regardless of their skill level. As the support rod 16 is manipulated, the position of the absorption body 15 with respect to the sheath 10 can be adjusted. As the distal end portion of the absorption body 15 is disposed closer to the distal end side than the sheath 10 when the absorption body 15 is pushed by the support rod 16, the absorption body 15 can easily come in contact with the duodenal juice P3.

In the embodiment, when the absorption body 15 absorbs the predetermined amount of the duodenal juice P3, the distal end portion of the absorption body 15 protrudes from the sheath 10 and only the proximal end portion of the absorption body 15 is disposed in the lumen 11. However, when the locked section 17 is locked to the locking section 13 while the absorption body 15 is in the initial state or when the absorption body 15 has absorbed the predetermined amount of the duodenal juice P3, the entire absorption body 15 may be disposed in the lumen 11. This is because, even when the entire absorption body 15 in the initial state is disposed in the lumen 11, the duodenal juice P3 entering the lumen 11 from the opening of the distal end side can be absorbed by the absorption body 15. In addition, as the entire absorption body 15 that has absorbed the predetermined amount of the duodenal juice P3 is disposed in the lumen 11, the entire absorption body 15 can be prevented from coming in contact with the inner circumferential surface of the channel 219 and thus the recovery becomes easier.

The configurations of the device 1 according to the embodiment can be variously modified as will be described below.

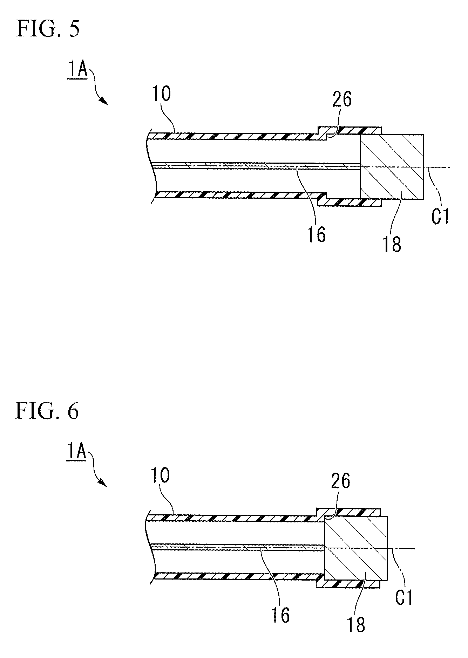

As a device 1A shown in Fig. 5, a stepped portion 26 may be formed at the proximal end side of the sheath 10 by increasing a diameter thereof. The inner diameter of the lumen 11 closer to the distal end than the stepped portion 26 is smaller than the outer diameter of the handle 18. In the example, the locking section 13 and the locked section 17 are not provided in the device 1A. As shown in Fig. 6, in the device 1A having the afore-mentioned configuration, when the handle 18 is pushed with respect to the sheath 10, the handle 18 is locked to the stepped portion 26 of the sheath 10. Even when the device 1 A is configured as described above, the position along the longitudinal axis C1 of the absorption body 15 with respect to the sheath 10 can be determined.

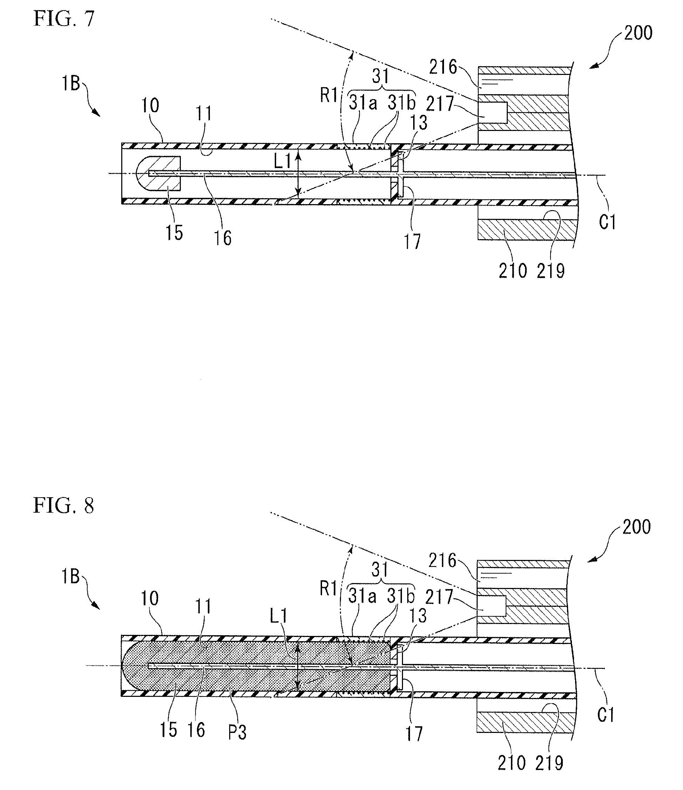

As a device 1B shown in Fig. 7, a color change section 31 may be provided instead of the obscured glass 20. The color change section 31 contains a pigment 31b of Commelina communis (Asiatic dayflower) serving as the identification section at an inner circumferential surface side of a sheet glass 31 a. The pigment 31 b is violet when not in contact with the duodenal juice P3. The pigment 31b can be observed by the observation unit 217 of the endoscope 200 from the outer circumferential surface side of the sheath 10 as the sheet glass 31 a allows penetration of the illumination light.

The outer diameter of the absorption body 15 in the initial state is smaller than the inner diameter L1 of the lumen 11. When the locked section 17 is locked to the locking section 13, the entire absorption body 15 in the initial state is positioned to be disposed in the lumen 11 closer to the distal end side than the color change section 31. That is, in this state, the absorption body 15 does not come in contact with the pigment 31 b.

When the absorption body 15 of the device 1B having the afore-mentioned configuration absorbs the predetermined amount of the duodenal juice P3, as shown in Fig. 8, the outer diameter of the absorption body 15 that has absorbed the duodenal juice P3 to be expanded becomes equal to the inner diameter L1 of the lumen 11, and the proximal end of the absorption body 15 extends to be more proximal than the distal end of the area in which the pigment 31b is provided. When the proximal end portion of the absorption body 15 comes in contact with the pigment 31b, the color of the pigment 31b is changed from violet to colorless as the pigment 31b reacts with moisture contained in the duodenal juice P3. The user who has observed the change of the color using the endoscope 200 can identify that the absorption body 15 has absorbed the predetermined amount of the duodenal juice P3. In the modified example, since the entire absorption body 15 is positioned in the distal end portion of the lumen 11, the absorption body 15 that has absorbed the duodenal juice P3 can be prevented from coming in contact with the inner circumferential surface of the channel 219 or the like, and thus the absorption body 15 can be more easily recovered.

Further, in the modified example, the entire color change section may be configured to solidify the pigment of Commelina communis by a known means.

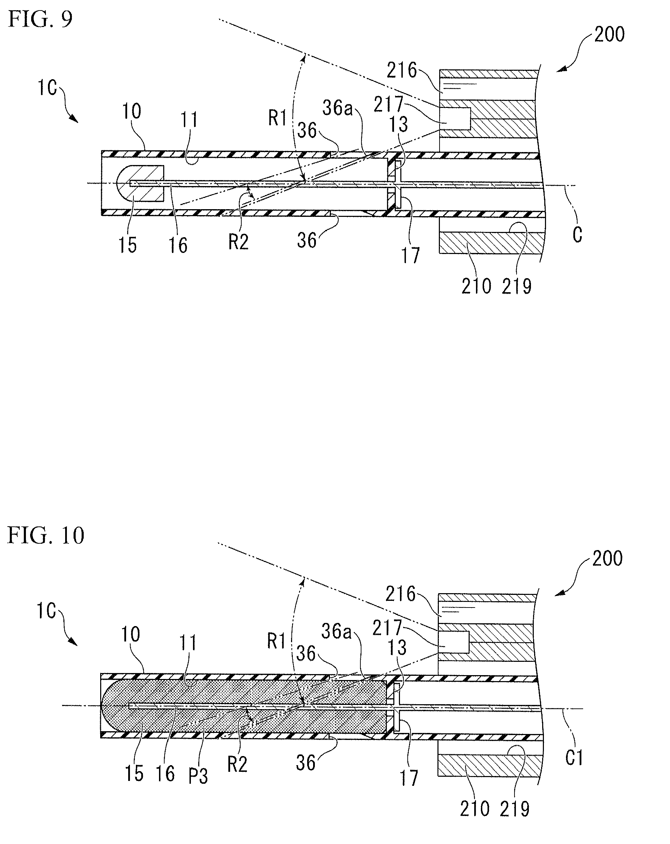

As a device 1C shown in Fig. 9, instead of the obscured glass 20 serving as the identification section, a side hole 36 formed to reach the lumen 11 from the outer circumferential surface of the sheath 10 may be provided. It is preferable that a surface 36a of the proximal end side of the side hole 36 is inclined to be spaced apart from the longitudinal axis C1 as it approaches the proximal end side so that the inside of the side hole 36 can be easily observed from the proximal end side outside the side hole 36. The side hole 36 may be solely formed in the sheath 10, or the plurality of side holes 36 may be formed to be separate from each other around the longitudinal axis C1. The absorption body 15 in the initial state when the locked section 17 is locked to the locking section 13 and positioned thereat is disposed at a position at which the side hole 36 is not blocked. Specifically, the absorption body 15 is disposed closer to the distal end side than the side hole 36, and more specifically, disposed closer to the distal end side than a range R2 in the sheath 10 that can be observed through the side hole 36 in the field of vision range R1. The absorption body 15 cannot be observed from the observation unit 217 of the endoscope 200 through the side hole 36 because the absorption body 15 is blocked by a wall section of the sheath 10.

When the absorption body 15 of the device 1C having the above-mentioned configuration absorbs the predetermined amount of the duodenal juice P3, as shown in Fig. 10, the absorption body 15 that expands to extend toward the proximal end side blocks the side hole 36. As a portion of the absorption body 15 is disposed in the range R2, the absorption body 15 can be observed through the side hole 36 by the observation unit 217. The same effect as the device 1 according to the embodiment can be exhibited by the device 1C of the modified example.

### (Second embodiment)

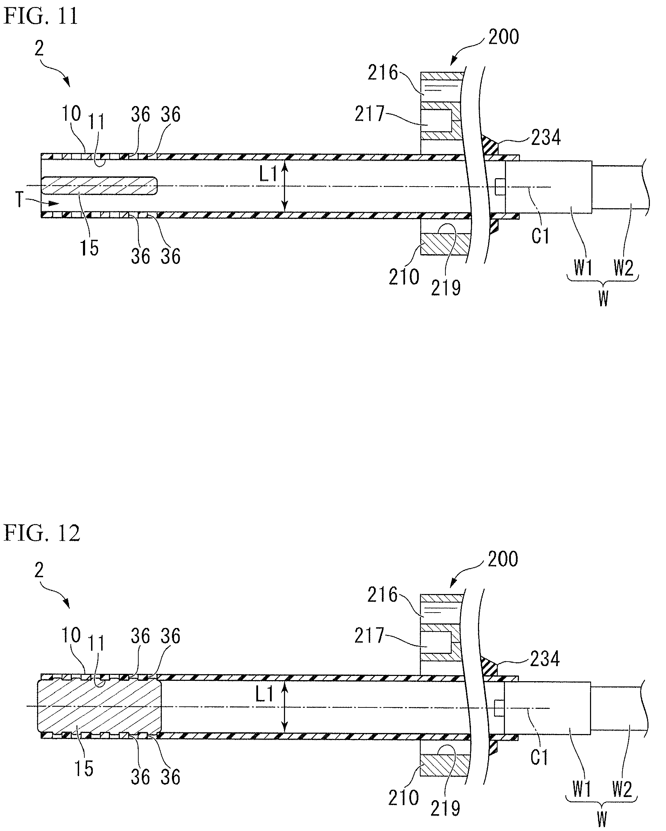

Next, a second embodiment of the present invention will be described with reference to Figs. 11 and 12. Parts that are the same as those of the previous embodiment are designated by the same reference numerals, description thereof will be omitted, and only different points will be described.

As shown in Fig. 11, a device 2 according to the embodiment includes the above-mentioned side hole 36, instead of the locking section 13, the support rod 16, the locked section 17, and the handle 18 of the device 1 according to the first embodiment. In the example, the plurality of side holes 36 are formed in the sheath 10 such that the plurality of side holes 36 are arranged to be separate from each other in a direction along the longitudinal axis C1. While not shown, the plurality of side holes 36 are also formed in the radial direction of the sheath 10. The absorption body 15 in the initial state is positioned such that the entire absorption body 15 is disposed in the distal end portion of the lumen 11, and supported at the sheath 10 by a support member (not shown). The outer diameter of the absorption body 15 in the initial state is smaller than the inner diameter L1 of the lumen 11. The absorption body 15 is separate from the side holes 36 in the lumen 11 to be disposed inside in the radial direction with respect to the side holes 36. That is, a gap T is formed between the inner circumferential surface of the lumen 11 and the absorption body 15 throughout the entire circumference of the absorption body 15. The absorption body 15 in the initial state does not completely block the lumen 11. The absorption body 15 in the initial state forms the gap T between the lumen 11 and the absorption body 15 and does not block the side hole 36.

The device 2 having the above-mentioned configuration is used in combination with the endoscope 200 as described above. The sheath 10 is inserted into the channel 219 through the forceps port 234. A syringe main body W1 of a known syringe W serving as the suction mechanism is attached to the proximal end portion of the sheath 10 of the device 2 in an air-tight manner with respect to the sheath 10. The distal end portion of the sheath 10 protrudes from the channel 219, and the sheath 10 is inserted into the duodenum P2 (not shown). The user grips the syringe W to pull a plunger W2 with respect to the syringe main body W1. The inside of the lumen 11 has a negative pressure lower than the inside of the duodenum P2, and the gas or the duodenal juice P3 in the body is pulled into the lumen 11 through the gap T of the lumen 11 or the side holes 36. When the duodenal juice P3 is absorbed, the absorption body 15 expands outward in the radial direction as shown in Fig. 12, and when the predetermined amount of the duodenal juice P3 is absorbed, the outer diameter of the absorption body 15 is equal to the inner diameter L1 of the lumen 11. Accordingly, the expanded absorption body 15 blocks the opening of the distal end side of the lumen 11 and the side holes 36. Since the gas or the like is hard to be pulled into the lumen 11 through the opening or the side holes 36, it is hard for the user to pull the plunger W2 back, and the user identifies that the absorption body 15 has absorbed the predetermined amount of the duodenal juice P3 by a magnitude of a force required for pulling the plunger W2 back, i.e., a feeling in his or her hand.

As described above, according to the device 2 of the embodiment, the absorption body 15 can be easily recovered in a state in which the predetermined amount of the duodenal juice P3 is absorbed into the absorption body 15. In addition, since there is no need to observe through the monitor 245 that the absorption body 15 has absorbed the predetermined amount of the duodenal juice P3, the user can observe portions other than the absorption body 15 using the monitor 245. Further, while the plurality of side holes 36 are formed in the sheath 10 in the embodiment, only one side hole 36 may be formed in the sheath 10.

### (Third embodiment)

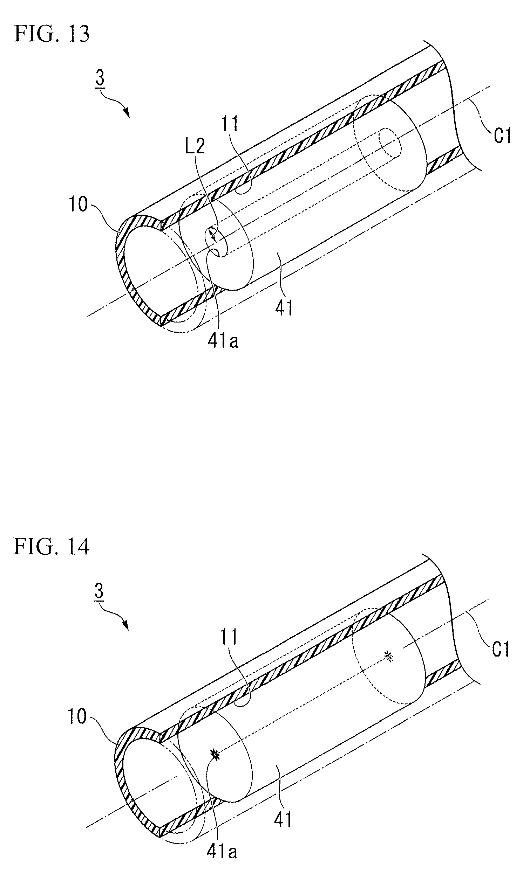

Next, a third embodiment of the present invention will be described with reference to Figs. 13 and 14. Parts that are the same as those of the previous embodiment are designated by the same reference numerals, description thereof will be omitted, and only different points will be described.

As shown in Fig. 13, a device 3 according to the embodiment includes an absorption body 41 instead of the absorption body 15 and the plurality of side holes 36 of the device 2 according to the second embodiment. The absorption body 41 is formed in a columnar shape. The outer circumferential surface of the absorption body 41 comes in contact with the inner circumferential surface of the lumen 11 throughout the entire circumference. A communication hole (an identification section) 41 a having a radius L2 is formed in the absorption body 41 in the initial state along the longitudinal axis C1. The absorption body 41 may be formed of the same material as the above-mentioned absorption body 15.

When the device 3 having the above-mentioned configuration is used, the syringe main body W1 is attached to the proximal end portion of the sheath 10, and the sheath 10 is inserted into the duodenum P2. When the plunger W2 is pulled back, the gas in the body is pulled into the lumen 11 through the communication hole 41 a of the absorption body 41. The absorption body 41 expands by absorbing the duodenal juice P3, and thus, as shown in Fig. 4, the inner circumferential surface of the communication hole 41 a expands inward in the radial direction throughout the entire circumference. When the absorption body 41 absorbs the predetermined amount of the duodenal juice P3, the inner circumferential surface of the communication hole 41 a expands from the initial state by the radius L2. For this reason, the communication hole 41a is blocked, and it is hard to pull the gas into the lumen 11 through the communication hole 41a. As the user cannot easily pull the plunger W2 back, the user identifies that the absorption body 41 has absorbed the predetermined amount of the duodenal juice P3.

As described above, according to the device 3 of the embodiment, the same effect as the device 2 of the second embodiment can be exhibited.

### (Fourth embodiment)

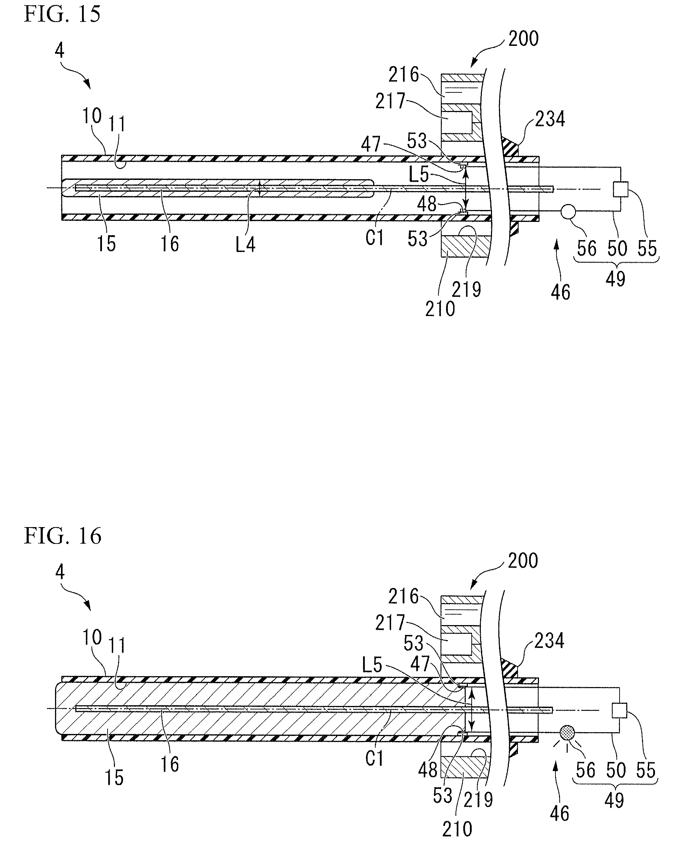

Next, a fourth embodiment according to the present invention will be described with reference to Figs. 15 and 16. Parts that are the same as those of the previous embodiment are designated by the same reference numerals, description thereof will be omitted, and only different points will be described.

As shown in Fig. 15, a device 4 according to the embodiment includes an identification section 46 instead of the locking section 13, the locked section 17, and the handle 18 of the device 1 according to the first embodiment. The identification section 46 has a pair of electrodes 47 and 48 provided at the inner circumferential surface of the lumen 11, and a light generating section 49 connected to the pair of electrodes 47 and 48.

In the example, the electrodes 47 and 48 are attached to the inner circumferential surface of the lumen 11 via an insulating member 53. The electrodes 47 and 48 are disposed to face each other. The light generating section 49 has a wiring 50 having end portions connected to the respective electrodes 47 and 48, and a DC power supply 55 such as a battery that are provided at the wiring 50 in series, and a warning lamp 56. While not shown, the wiring 50 is configured by coating an electric wire having conductivity with a coating material having insulation. The wiring 50 has the electric wires disposed at the respective end portions and exposed from the coating material, and the electric wires are electrically connected to the respective electrodes 47 and 48. The DC power supply 55 and the warning lamp 56 have known configurations, and are electrically connected to the electric wires of the wiring 50. The outer diameter L4 of the absorption body 15 in the initial state is equal to or smaller than a distance L5 between the electrodes 47 and 48. In the initial state, the absorption body 15 is not disposed between the electrodes 47 and 48.

In the device 4 having the above-mentioned configuration, when the sheath 10 of the device 4 is inserted through the forceps port 234, the warning lamp 56 is preferably disposed in the vicinity of the user. As the duodenal juice P3 is absorbed, the absorption body 15 expands to extend toward the proximal end side while the outer diameter increases. When the absorption body 15 absorbs the predetermined amount of the duodenal juice P3, as shown in Fig. 16, the outer diameter is increased to be equal to or larger than the above-mentioned distance L5 while the proximal end portion of the absorption body 15 is disposed between the electrodes 47 and 48. Accordingly, the expanded absorption body 15 comes in contact with each of the electrodes 47 and 48. Since the absorption body 15 has conductivity when it absorbs the duodenal juice P3 containing moisture, the electrode 47 and the electrode 48 are electrically connected to each other via the absorption body 15. A closed circuit is constituted by the electrodes 47 and 48, the light generating section 49, and the absorption body 15, and current flows to the wiring 50 from the DC power supply 55. The warning lamp 56 emits light that arouses caution in the user, and the user identifies that the absorption body 15 has absorbed the predetermined amount of the duodenal juice P3.

As described above, according to the device 4 of the embodiment, the absorption body 15 can be easily recovered in a state in which the predetermined amount of the duodenal juice P3 is absorbed into the absorption body 15. Even when a field of vision of the observation unit 217 is bad because of adhesion of the body fluid to the portion of the observation unit 217 exposed to the outside, absorption of the predetermined amount of the duodenal juice P3 by the absorption body 15 can be more reliably identified by the light emitted from the warning lamp 56.

In the embodiment, the identification section 46 includes the light generating section 49 configured to emit light. However, the identification section may include a sound generating section having a speaker or the like configured to generate sound, or may include a vibration generating section having a vibrator or the like configured to generate vibrations, instead of the light generating section 49. In this case, the user can identify that the absorption body 15 has absorbed the predetermined amount of the duodenal juice P3 through a sense other than vision.

When insulation of the sheath 10 is high, the electrodes 47 and 48 may be directly attached to the inner circumferential surface of the lumen 11 without intervention of the insulating member 53. In the initial state, the absorption body 15 may be disposed between the electrodes 47 and 48. In this case, as the outer diameter L4 of the absorption body 15 is smaller than the distance L5 between the electrodes 47 and 48, the absorption body 15 is configured not to come in simultaneous contact with the electrodes 47 and 48.

Hereinabove, while the first embodiment to the fourth embodiment of the present invention have been described with reference to the accompanying drawings, the specific configurations are not limited to the embodiments and may include modifications, combinations, or the like without departing from the scope of the present invention. Further, the configurations described in the embodiments may be used in combination.

For example, in the first embodiment to the fourth embodiment, while the body fluid is the duodenal juice P3, the body fluid is not limited thereto and may be, for example, pancreatic juice, bile, or the like. In the second embodiment and the fourth embodiment, both before the duodenal juice P3 is absorbed and after the predetermined amount of the duodenal juice P3 is absorbed, the entire absorption body 15 may be disposed in the lumen 11. According to the above-mentioned configuration, the shape of the absorption body 15 after absorption of the duodenal juice P3 and expansion is further stabilized, and precision of the amount of the duodenal juice P3 absorbed can be increased. While the case in which the device is used in combination with the direct viewing type endoscope 200 has been described, the devices may be used in combination with a so-called side viewing type endoscope.

The present invention is not limited by the above-mentioned description, and is limited only by the scope of the accompanying claims.

### Industrial Applicability

According to the body fluid collection device of each of the embodiments, the absorption body can be easily recovered in a state in which the predetermined amount of the body fluid is absorbed into the absorption body.

### Reference Signs List

- 1, 1A, 1B, 1C, 2, 3, 4:: device (body fluid collection device)
- 10:: sheath (long shaft member)
- 11:: lumen
- 15, 41:: absorption body
- 16:: support rod (manipulation member)
- 20:: obscured glass (identification section)
- 21:: obscured machined surface
- 31b:: pigment (identification section)
- 36:: side hole
- 41a:: communication hole (identification section)
- 46:: identification section
- 47, 48:: electrode
- 49:: light generating section
- C1:: longitudinal axis
- P3:: duodenal juice (body fluid)

## Claims

1. A body fluid collection device comprising:
a long shaft member having a lumen formed along a longitudinal axis;
an absorption body configured to expand from an initial state in which a body fluid is not absorbed into the absorption body as the body fluid is absorbed, and having a proximal end portion configured to be disposed in the lumen when a predetermined amount of the body fluid is absorbed into the absorption body; and
an identification section for identifying that the absorption body has absorbed the predetermined amount of the body fluid.

2. The body fluid collection device according to claim 1, wherein
the identification section is an obscured glass provided at the long shaft member such that the obscured glass is observable from an outer circumferential surface side, and a surface of the obscured glass disposed at an inner circumferential surface side of the long shaft member is an obscured machined surface,
the absorption body has an outer diameter equal to or smaller than an inner diameter of the lumen in the initial state, and is disposed more distal than the obscured machined surface; and
when the absorption body has absorbed the predetermined amount of the body fluid, the outer diameter of the absorption body becomes equal to the inner diameter of the lumen, and a proximal end of the absorption body extends to be closer to a proximal end of the long shaft member than a distal end of the obscured machined surface.

3. The body fluid collection device according to claim 2, wherein
the absorption body is attached to a distal end portion of a manipulation member inserted into the lumen such that the manipulation member is movable along the longitudinal axis, and
when the manipulation member is moved to the distal end side with respect to the long shaft member, a distal end portion of the absorption body is disposed more distal than the long shaft member.

4. The body fluid collection device according to claim 1, wherein
the identification section is a pigment of Commelina communis provided at an inner circumferential surface side of the long shaft member such that the pigment is observable from an outer circumferential surface side of the long shaft member,
the absorption body is disposed more distal than the pigment in the initial state,
and
when the absorption body has absorbed the predetermined amount of the body fluid, an outer diameter of the absorption body becomes equal to an inner diameter of the lumen, and a proximal end of the absorption body extends to be closer to a proximal end of the long shaft member than a distal end of an area in which the pigment is provided.

5. The body fluid collection device according to claim 1, wherein
the identification section is a side hole formed to reach the lumen from an outer circumferential surface of the long shaft member,
the absorption body does not block the side hole in the initial state, and
when the absorption body has absorbed the predetermined amount of the body fluid, the expanded absorption body blocks the side hole.

6. The body fluid collection device according to claim 1, wherein
the identification section is a side hole formed to reach the lumen from an outer circumferential surface of the long shaft member,
the absorption body has an outer diameter smaller than an inner diameter of the lumen in the initial state, and is disposed inside the lumen to be spaced from the side hole and disposed radially inward with respect to the side hole, and
when the absorption body has absorbed the predetermined amount of the body fluid, the absorption body expands radially outward such that the outer diameter of the absorption body becomes equal to the inner diameter of the lumen.

7. The body fluid collection device according to claim 1, wherein
the absorption body has an outer circumferential surface in contact with an inner circumferential surface of the lumen throughout an entire circumference of the absorption body,
the identification section is a communication hole formed in the absorption body along the longitudinal axis, and
when the absorption body has absorbed the predetermined amount of the body fluid, an inner circumferential surface of the communication hole of the absorption body expands radially inward by a radius of the communication hole.

8. The body fluid collection device according to claim 1, wherein
the identification section has:
a pair of electrodes provided on an inner circumferential surface of the lumen to face each other; and
a light generating section, a sound generating section, or a vibration generating section connected to each of the electrodes and configured to be operated when a closed circuit is formed together with the electrodes,
the absorption body has an outer diameter equal to or smaller than a distance between the electrodes in the initial state,
in the initial state, the absorption body is not disposed between the electrodes, and
when the absorption body has absorbed the predetermined amount of the body fluid, the expanded absorption body is disposed between the electrodes, and the outer diameter of the absorption body is increased to be equal to or larger than the distance between the electrodes.
